(19) Europäisches Patentamt
European Patent Office
Office européen des brevets

(11) **EP 4 752 527 A1**

(12) **EUROPEAN PATENT APPLICATION**

(43) Date of publication:
**03.06.2026 Bulletin 2026/23**

(21) Application number: **25217456.0**

(22) Date of filing: **20.11.2025**

(51) International Patent Classification (IPC):
**G01N 15/0227** *(2024.01)* **G01N 15/1429** *(2024.01)*
**G01N 15/1434** *(2024.01)* **G01N 21/552** *(2014.01)*
**G01N 21/64** *(2006.01)* **G01N 33/50** *(2006.01)*
**G01N 33/58** *(2006.01)*

(52) Cooperative Patent Classification (CPC):
**G01N 15/1433; G01N 15/1434; G01N 21/6458;
G01N 21/648; G01N 33/5076; G01N 33/582;
G01N 33/587; G01N 33/588;** G01N 2015/0038;
G01N 2015/1006

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **27.11.2024 JP 2024205909**

(71) Applicant: **Canon Kabushiki Kaisha
Tokyo, 146-8501 (JP)**

(72) Inventors:
• **EGUCHI, Akira
Tokyo, 146-8501 (JP)**
• **YAMAUCHI, Fumio
Tokyo, 146-8501 (JP)**
• **SUGAWA, Yoshihiko
Tokyo, 146-8501 (JP)**

(74) Representative: **Canon Europe Limited
European Intellectual Property Group
4 Roundwood Avenue
Stockley Park
Uxbridge UB11 1AF (GB)**

(54) **MEASUREMENT METHOD, MEASUREMENT APPARATUS, AND PROGRAM**

(57) A measurement method includes measuring fluorescent light emitted from fluorescent dyes (2002) of each of a plurality of fine particles (2001) that are bound with the fluorescent dyes, and measuring reference light emitted from each of the plurality of fine particles, acquiring a ratio of particles for which both the fluorescent light from the fluorescent dyes and the reference light are detected in the plurality of fine particles, and acquiring a statistical value of a fluorescence amount of the fluorescent dyes based on the fluorescence amount of the fluorescent dyes and the ratio of the particles.

FIG. 5

## Description

TECHNICAL FIELD

The present disclosure relates to one or more embodiments of a measurement method, a measurement apparatus, and a program.

BACKGROUND

Developments of measurement technologies for measuring weak fluorescent light emitted from microscopic specimens such as viruses and extracellular vesicles and observing each specimen are underway. Since each specimen can be measured, properties of not only a group of specimens but also an individual specimen can be analyzed, and the amount of obtained information significantly increases. Each of PCT Domestic Publication No. 2023-521872 and Jeong, Mi Ho, et al. "Plasmon-Enhanced Single Extracellular Vesicle Analysis for Cholangiocarcinoma Diagnosis." Advanced Science, Vol. 10, Issue 8, pp 2205148, January 2023, USA discloses a method for measuring a single extracellular vesicle by immobilizing extracellular vesicles on a plasmon substrate using an affinity ligand selectively bound to the extracellular vesicles, and by measuring fluorescent light amplified by plasmon resonance.

SUMMARY

The present disclosure in its first aspect provides a measurement method as specified in claims 1 to 18.
The present disclosure in its second aspect provides a measurement apparatus as specified in claim 19.
The present disclosure in its third aspect provides a program as specified in claim 20.
Features of the disclosure will become apparent from the following description of embodiments with reference to the attached drawings. The following description of embodiments will be provided by way of example.

BRIEF DESCRIPTION OF THE DRAWINGS

Fig. 1 is a schematic diagram of measurement apparatuses according to first to third embodiments.
Fig. 2 is a schematic view illustrating a measurement method according to a comparative example.
Fig. 3 illustrates an example of a measurement result in the comparative example.
Figs. 4A, 4B, 4C, and 4D are schematic diagrams of calibration particles in each embodiment.
Fig. 5 is a schematic view illustrating a measurement method according to each embodiment.
Fig. 6 is a flowchart illustrating the measurement method according to each embodiment.
Fig. 7 is a flowchart illustrating another measurement method according to each embodiment.
Fig. 8 is a schematic view illustrating a measurement method according to a variation of each embodiment.
Fig. 9 illustrates a measurement result in the first embodiment.
Fig. 10 illustrates a measurement result in the second embodiment.
Fig. 11 is a schematic diagram of a measurement apparatus according to a fourth embodiment.

DESCRIPTION OF THE EMBODIMENTS

In the following, the term "unit" may refer to a software context, a hardware context, or a combination of software and hardware contexts. In the software context, the term "unit" refers to a functionality, an application, a software module, a function, a routine, a set of instructions, or a program that can be executed by a programmable processor such as a microprocessor, a central processing unit (CPU), or a specially designed programmable device or controller. A memory contains instructions or programs that, when executed by the CPU, cause the CPU to perform operations corresponding to units or functions. In the hardware context, the term "unit" refers to a hardware element, a circuit, an assembly, a physical structure, a system, a module, or a subsystem. Depending on the specific embodiment, the term "unit" may include mechanical, optical, or electrical components, or any combination of them. The term "unit" may include active (e.g., transistors) or passive (e.g., capacitor) components. The term "unit" may include semiconductor devices having a substrate and other layers of materials having various concentrations of conductivity. It may include a CPU or a programmable processor that can execute a program stored in a memory to perform specified functions. The term "unit" may include logic elements (e.g., AND, OR) implemented by transistor circuits or any other switching circuits. In the combination of software and hardware contexts, the term "unit" or "circuit" refers to any combination of the software and hardware contexts as described above. In addition, the term "element," "assembly," "component," or "device" may also refer to "circuit" with or without integration with packaging materials.
Referring now to the accompanying drawings, a detailed description will be given of embodiments according to the disclosure. Each of the embodiments of the present disclosure described below can be implemented solely or as a combination of a plurality of the embodiments or features thereof where necessary or where the combination of elements or features from individual embodiments in a single embodiment is beneficial.

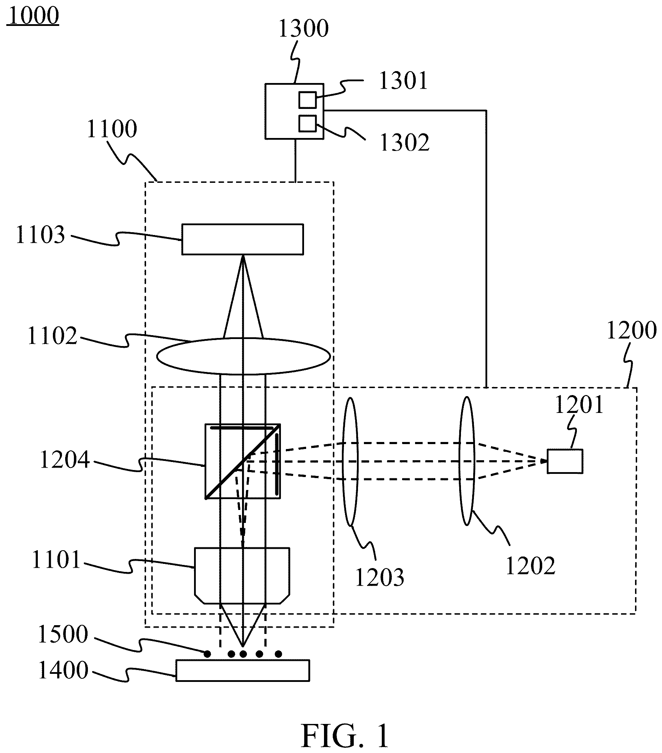

Fig. 1 is a schematic view illustrating the configuration of a fluorescence measurement apparatus 1000 according to each embodiment. The fluorescence measurement apparatus 1000 includes a microscope unit (detector) 1100, an illumination unit 1200, and a control unit 1300. A measurement unit includes the microscope unit 1100 and the illumination unit 1200. As described later, the measurement unit measures fluorescent light emitted from fluorescent dyes (fluorochromes) of each of a plurality of fine particles (or microparticles) that are labeled with the fluorescent dyes and emit reference light, and the reference light emitted from each of the plurality of fine particles. The fluorescence measurement apparatus 1000 irradiates a sample 1500 disposed on a substrate 1400 (chemically bound to the substrate) with illumination light and detects (measures) fluorescent light emitted from the sample 1500.

The microscope unit 1100 includes an optical system (measurement optical system) including an objective lens 1101 and an imaging lens 1102, and an image sensor 1103 such as a CMOS sensor. An enlarged image of the sample 1500 formed by the objective lens 1101 and the imaging lens 1102 is imaged by the image sensor 1103. To obtain images at different magnifications, the objective lens 1101 may be mounted on a revolver on which a plurality of objective lenses 1101 can be installed.

The illumination unit 1200 includes a light source 1201 and an illumination optical system. The illumination optical system includes a collimator lens 1202, a condenser lens 1203, and a filter cube 1204.

In the configuration illustrated in Fig. 1, the objective lens 1101 is shared by the microscope unit 1100 and the illumination unit 1200, and the illumination unit 1200 illuminates the sample 1500 with a configuration including the objective lens 1101.

The light source 1201 includes, for example, a light-emitting diode (LED) or a laser beam source but is not limited to this example. The light source 1201 may be constituted, for example, by combining a light source having a broad wavelength band, such as a halogen lamp or a white LED, with a proper band-pass filter. The illumination unit 1200 constitutes a Kohler illumination system using the collimator lens 1202, the condenser lens 1203, and the objective lens 1101.

The filter cube 1204 has a wavelength characteristic that reflects illumination light and transmits fluorescent light emitted from the sample 1500. The filter cube having such a wavelength characteristic may be configured, for example, by a combination of a band-pass filter that transmits only illumination light, a dichroic mirror that reflects only illumination light and transmits fluorescent light from the sample, and a band-pass filter that transmits only fluorescent light. As a simpler configuration, the filter cube 1204 may have a configuration combining a single band-pass filter and a dichroic mirror, or a configuration constituted by only a dichroic mirror. A plurality of filter cubes may be provided and switched in accordance with a target fluorescent dye. In this case, to facilitate switching, the filter cubes may be installed on a filter wheel that allows for selection of a filter cube to be used from among a plurality of filter cubes.

The control unit 1300 includes a dedicated computer or a personal computer and controls lighting of the light source of the illumination unit 1200, driving of an unillustrated drive mechanism, and image acquisition by the microscope unit 1100 in accordance with computer programs. More specifically, the control unit 1300 switches an illumination wavelength and the filter cube 1204 by communicating with the illumination unit 1200, and acquires a fluorescence image at each wavelength by communicating with the microscope unit 1100. In addition, the control unit 1300 may perform a calculation to estimate fluorescence intensity (fluorescence amount) and the amount of protein to be described later.

The control unit 1300 and each component may be directly connected to each other through a cable or the like, or may be connected to each other by using a short-distance communication system. The control unit 1300 may have functions to store images, perform calculations using images, display images, and the like, in addition to controlling the microscope unit 1100 and the illumination unit 1200. These functions may be achieved by another device through a network. Analyzing acquired images can provide information on proteins, RNA, and the like contained in the sample 1500.

The control unit 1300 includes a first acquiring unit 1301 and a second acquiring unit 1302. The first acquiring unit acquires the ratio (detection rate r) of particles for which both fluorescence of the fluorescent dye and the reference light are detected among a plurality of fine particles. The second acquiring unit 1302 calculates a statistical value (such as an average value) of the fluorescence amount based on the fluorescence amount and the ratio, as described later in detail.

The substrate 1400 is an element for enabling microscope observation of the sample 1500 immobilized on its surface and is, for example, a glass substrate, a substrate with a surface coated with dielectric or metal, or a plasmon substrate that causes plasmon resonance with a fine metal structure, but it is not limited to this example. A ligand (material specifically bound to a particular receptor) that selectively binds the sample 1500 onto the substrate may be provided on the surface of the substrate 1400.

The sample 1500 is a measurement target object and varies according to the purpose of measurement. In the evaluation and calibration of the measurement apparatus, fluorescent particles for calibration to be described later serve as the sample 1500. After the calibration is performed, for example, extracellular vesicles (such as exosomes) derived from biological tissue, viruses, or liposomes serve as the sample 1500. In each embodiment, fluorescent particles to be used as the sample 1500 during calibration will be referred to as calibration particles, and the sample 1500 to be measured after calibration will be referred to as a specimen.

In the specimen, the fluorescent dye is bound via an antibody corresponding to a target protein. When a fluorescence image of the specimen is acquired by the fluorescence measurement apparatus 1000, a plurality of light emission points are observed in the image in a case where the specimen contains the target protein. On the other hand, no light emission points are observed in the image in a case where the specimen does not contain the target protein. Each light emission point is generated by fluorescent light emitted from an individual specimen containing the target protein. Thus, it is possible to determine whether a specimen containing the target protein is present, according to the presence or absence of light emission points. A brighter light emission point indicates a greater amount of the target protein expressed in an individual specimen.

To quantify protein expressed in the specimen, the fluorescence intensity measured per fluorescent dye may be previously known. The measured fluorescence intensity is determined by a variety of factors including the intensity and wavelength of the light source 1201, the wavelength characteristic of the filter cube 1204, the sensitivity of the image sensor 1103, the wavelength characteristic and quantum efficiency of a fluorescent dye to be used, and deterioration over time of these elements. Thus, calibration using calibration particles may be periodically performed for an actual device to be used.

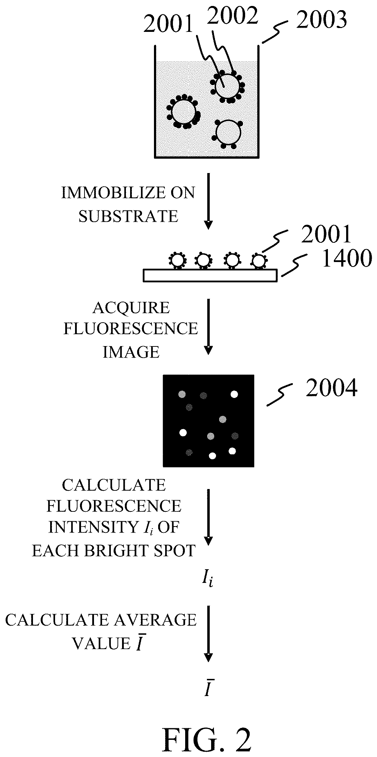

A calibration method (measurement method) according to a comparative example will be described below with reference to Fig. 2. Fig. 2 is a schematic view illustrating the measurement method according to the comparative example.

First, fluorescent particles (a plurality of fine particles) to which a fluorescent dye 2002 identical to a fluorescent dye to be bound to the specimen is bound are prepared as calibration particles 2001. The calibration particles 2001 are immobilized on the substrate 1400, and a fluorescence image 2004 is acquired by the fluorescence measurement apparatus 1000. The calibration particles 2001 may be as large as the specimen. In a case where extracellular vesicles, viruses, or the like are assumed as the specimen, their sizes are several tens to several hundreds of nanometers, and thus the calibration particles 2001 may have a size of several tens to several hundreds of nanometers.

In the acquired image, bright points (bright spots) having a size determined by the objective lens 1101 are observed at a plurality of places. Fluorescence intensity $I_i$ of the plurality of bright spots is extracted through image processing. A subscript i is a number of a bright spot and takes a value of 1 to N, where N is the number of bright spots in the image. The fluorescence intensity is a pixel value at each pixel in the output image in the following description, but is properly changed according to the specification of the fluorescent measurement apparatus. For example, in a case where a value output from the image sensor 1103 is a voltage value at each pixel, the voltage value is used as the fluorescence intensity; in a case where the output value is the amount of electric charge at each pixel, the amount of electric charge is used as the fluorescence intensity; or a value obtained by normalizing them to the bit depth of the image is used as the fluorescence intensity. In a case where an avalanche photodiode (APD) or a photomultiplier is used as the image sensor 1103, a signal value such as current output from a detector, is used as the fluorescence intensity. A value output from the fluorescent measurement apparatus in accordance with the amount of fluorescent light reaching a light detecting element or detector is used as the fluorescence intensity. The fluorescence intensity has a correspondence relationship with the amount of fluorescent light reaching the detector and the amount of fluorescent light emitted from the sample, and thus whether the fluorescence intensity or the fluorescence amount is to be analyzed is not particularly distinguished.

On the other hand, the number of fluorescent dyes 2002 bound to the calibration particles 2001 is measured. This can be calculated from measurement results of the absorbance of a solution and the particle number concentration. Normally, since the calibration particles 2001 are dispersed in the solution, absorbance A of the solution 2003 can be measured. This may be measured by using a general spectrophotometer. The absorbance A is expressed by the following equation (1) using a molar absorption coefficient $\varepsilon$ of the fluorescent dye, a dye concentration $c_A$, and a thickness L of a container holding the solution:

$$A = \varepsilon c_A L \qquad \dots (1)$$

The molar absorption coefficient $\varepsilon$ is generally known. Thus, by using equation (1), the dye concentration $c_A$ can be obtained by equation (2) below:

$$c_A = \frac{A}{\varepsilon L} \qquad \dots (2)$$

In addition to the absorbance measurement, the number (particle number concentration) $c_p$ of particles per unit volume of the solution 2003 containing the calibration particles 2001 is measured. This can be measured by a general dynamic scattering method or a nanoparticle tracking analysis method. Alternatively, this can be calculated by comparing the absorption spectrum of a solution with a known concentration and the absorption spectrum of the solution 2003 containing the calibration particles.

By calculating a ratio of $c_A$ and $c_p$ obtained earlier, an average value $\overline{x_0}$ of the number of dyes bound per particle can be found from equation (3) below:

$$\bar{x}_0 = \frac{c_A}{c_p} \qquad \dots (3)$$

Finally, an average value $\bar{I}$ of the fluorescence intensity is divided by $\overline{x_0}$. Accordingly, as expressed by equation (4) below, fluorescence intensity (conversion coefficient) $\gamma$ corresponding to the fluorescence amount per dye of the fluorescent dye can be obtained:

$$\gamma = \frac{\bar{I}}{\bar{x}_0} \qquad \cdots (4)$$

After the calibration is performed, a fluorescence image of the specimen is acquired by using the fluorescence measurement apparatus 1000. As in the fluorescence image 2004, a large number of bright spots are observed in the obtained fluorescence image. The fluorescence intensity $I_i$ of bright spots is extracted through image processing in a manner similar to that in the calibration and divided by the conversion coefficient $\gamma$ obtained during the calibration. Thereby, the number (dye amount) $x_i$ of dyes bound per specimen can be obtained as expressed by equation (5) below:

$$x_i = \frac{I_i}{\gamma} \qquad \cdots (5)$$

This method can be performed by using a variety of known measurement methods and publicly known information, but it has the following problems. Since fluorescent light emitted from a microscopic specimen such as an extracellular vesicle or virus, which is several tens to several hundreds of nanometers is weak, a highly sensitive fluorescence measurement method is demanded. In order to improve the sensitivity, one conceivable method is to immobilize a specimen on a plasmon substrate or a metal-coated substrate, and observe it as disclosed in PCT Domestic Publication No. 2023-521872. However, the light emission intensity varies on such a substrate according to the size and refractive index of the specimen and the dye binding method. That is, the conversion coefficient $\gamma$ can vary according to the specimen. Thus, the calibration may use fluorescent particles that simulate the size and refractive index of the specimen to be measured, the dye binding method, and the like. In this case, the calibration particles having a size of several tens to several hundreds of nanometers approximately may be prepared, and fluorescence is weak even for the calibration particles.

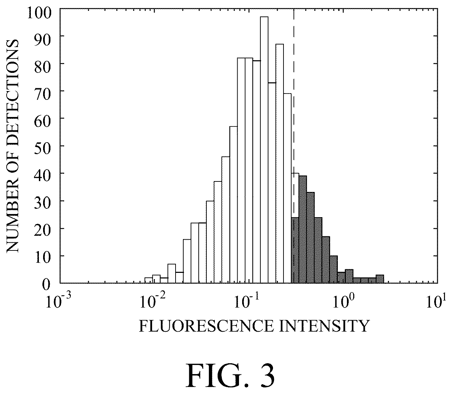

Referring now to Fig. 3, a description will be given of problems when the fluorescence intensity of each bright spot is measured by using the calibration particles. Fig. 3 illustrates a measurement result example in a comparative example. In Fig. 3, the horizontal axis represents the fluorescence intensity, and the vertical axis represents the number of detections (detection count).

Since the number of dyes bound to a particle is different for each particle, the fluorescence intensity $I_i$ is not uniform and exhibits variation as indicated by the white histogram in Fig. 3. Since the fluorescence measurement apparatus 1000 has a detection limit, it cannot observe particles that emit only fluorescent light lower than the detection limit indicated by a broken line in Fig. 3. In a case where particles are small, there exist a large number of particles that emit only a fluorescence amount below the detection limit. Thus, bright spots that can be detected in the image are only those with high fluorescence intensity, which are indicated by a black histogram in Fig. 3. When the fluorescence intensities $I_i$ of such bright spots are averaged, a value larger than the true average value for all particles is obtained. In a case where the overestimated average value is used, the conversion coefficient $\gamma$ of equation (4) described above is overestimated, and as a result, an error occurs in the number of dyes $x_i$ of the specimen estimated by equation (5).

From the above, one of the purposes of each embodiment is to correctly estimate an average value $\bar{I}$ even when there are calibration particles that have low fluorescence intensity and are not detected. Another purpose of each embodiment is to correctly estimate an amount $y_i$ of protein in each specimen by obtaining the conversion coefficient $\gamma$ using the correctly estimated average value $\bar{I}$. In order to solve these problems, each embodiment uses, as calibration particles, particles having a size comparable to that of the specimen, to which evaluation fluorescent dyes are bound and emitting sufficiently bright reference light.

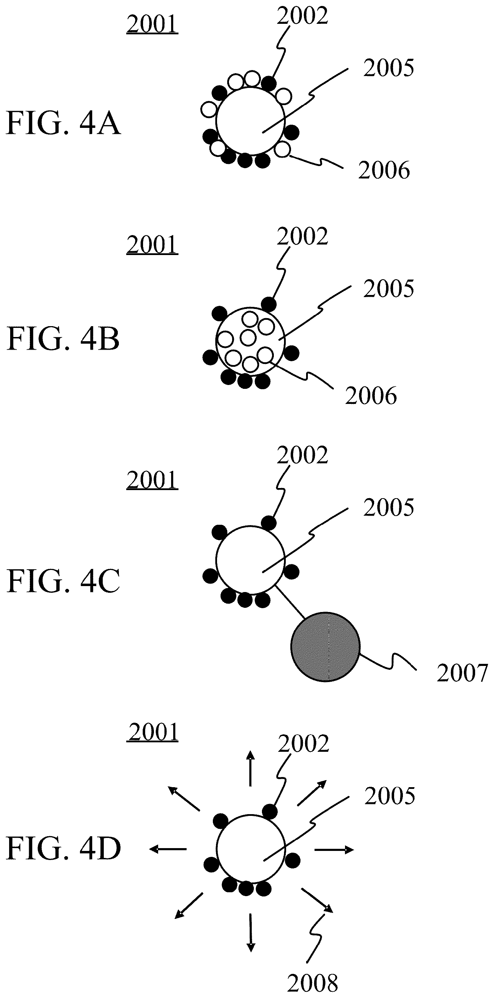

The calibration particles 2001 will be described with reference to Figs. 4A, 4B, 4C, and 4D. Figs. 4A, 4B, 4C, and 4D are schematic diagrams of the calibration particles 2001 according to each embodiment. The material of a particle 2005 to which fluorescent dyes are bound can use general particle material, such as silica or polystyrene. Since a biologically derived substance has a small refractive index, silica may be used as the material. The size of the calibration particles 2001 may be equivalent to that of the specimen. In a case where, for example, extracellular vesicles or viruses are assumed as the specimen, the diameter of the particle 2005 is desirably 10 nm to 500 nm, or 40 nm to 200 nm.

The evaluation fluorescent dye 2002 is a fluorescent dye with which the fluorescence measurement apparatus 1000 is calibrated. The evaluation fluorescent dye 2002 may be the same as a fluorescent dye that is used when the specimen is measured. The fluorescence dye 2002 and the particle 2005 can be bound by general chemical reaction such as amide bonding or antibody reaction, but a method that is the same method for binding the specimen and the evaluation fluorescent dye 2002 may be used. For example, in a case where an evaluation fluorescent dye is bound to the specimen via an antibody, the particle 2005 and the evaluation fluorescent dye 2002 may be bound via the antibody as well. In a case where the specimen and the fluorescent dye 2002 are bound via a plurality of antibodies, the particle 2005 and the evaluation fluorescent dye 2002 may be bound via the plurality of antibodies in a similar manner. In a case where the target protein in the specimen is a protein expressed on a membrane of the specimen, the evaluation fluorescent dye 2002 may be bound on the surface of the particle 2005. In a case where the target protein is a protein expressed inside the specimen, the evaluation fluorescent dye 2002 may be encapsulated in the particle 2005.

Light for reference (reference light) is fluorescent light from a fluorescent dye having absorption and emission wavelengths different from those of the evaluation fluorescent dye 2002. For example, the wavelength of the reference light is shorter than that of light from the fluorescent dye. The fluorescent light emitted from an evaluation fluorescent dye can be prevented from being absorbed by the reference fluorescent dye. However, each embodiment is not limited to this example. Each example excellently functions by binding a fluorescent dye having such a wavelength characteristic to the particle 2005 as a reference fluorescent dye 2006 as illustrated in Fig. 4A. Not only fluorescent dyes but also light emission materials such as quantum dots may be bound to the particle.

As illustrated in Fig. 4B, the reference fluorescent dye 2006 may be encapsulated in the particle 2005. Encapsulating the reference fluorescent dye 2006 in the particle 2005 can have a larger number of dyes than those bound to the surface, thereby providing more reference fluorescence. As illustrated in Fig. 4C, a metal nanoparticle 2007 may be bound to the particle 2005. The metal nanoparticle 2007 emits strong scattered light, and thus the scattered light from the metal nanoparticle 2007 can be used as the reference light. As illustrated in Fig. 4D, scattered light 2008 emitted from the particle 2005 may be used as light for reference (reference light). The reference light is used to specify the positions and number of calibration particles as described later, and thus any light that allows the specification can be used as the reference light. The following description will be made with the calibration particle illustrated in Fig. 4A as an example.

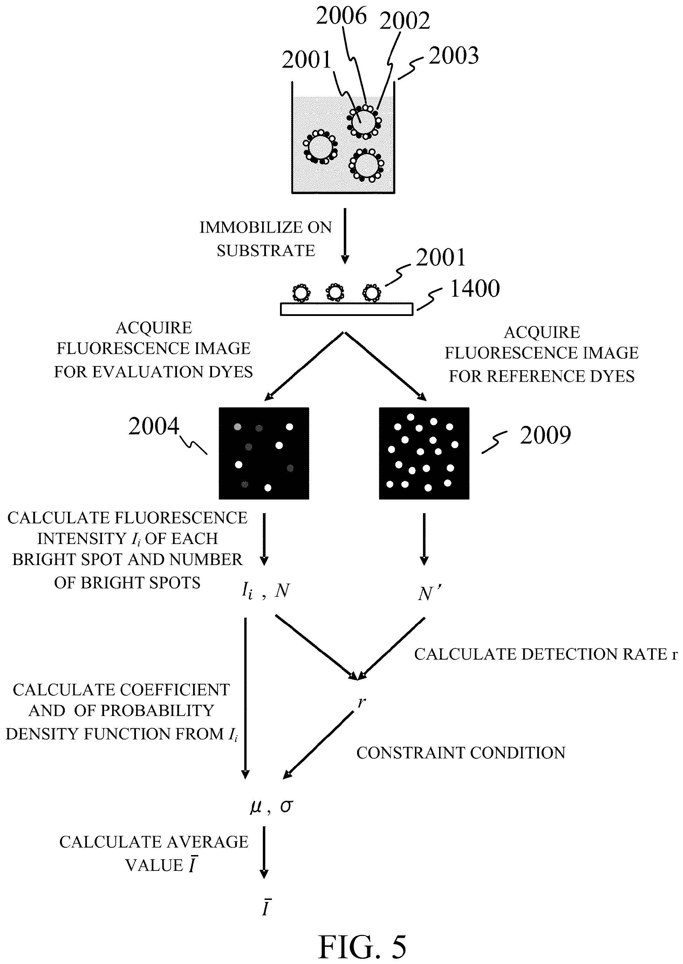

Referring now to Fig. 5, a description will be given of a method for calculating an average value of the fluorescence intensity of the evaluation dye measured by the fluorescence measurement apparatus 1000 using the calibration particles 2001. Fig. 5 is a schematic view illustrating the measurement method according to each embodiment.

The solution 2003 in which the calibration particles 2001 are dispersed is dropped onto the substrate 1400, and the calibration particles 2001 are immobilized on the substrate 1400. The immobilizing method is not particularly limited but may be the same as a method for immobilizing the specimen onto the substrate. For example, in a case where the specimen and the substrate are chemically bound by via a ligand, the calibration particles may be immobilized to the substrate using the same ligand. In a case where a solution containing the specimen is dried for immobilization, the calibration particles 2001 may be immobilized by drying in a similar manner.

The control unit 1300 in the fluorescence measurement apparatus 1000 acquires fluorescence images of the immobilized fluorescent particles. The fluorescence images are two captured images: a fluorescence image (first image) 2004 for the evaluation fluorescent dye 2002 and a fluorescence image (second image) 2009 for the reference fluorescent dye 2006 (the reference light). The measurement order is not limited, and the measurement may be simultaneously performed.

In each image, bright spots having a size determined by the objective lens 1101 are observed at a plurality of places. Since evaluation fluorescent light and reference fluorescent light are emitted from the same particle, the bright spots exist at substantially the same positions in the two fluorescence images 2004 and 2009. These bright spots are detected from the two images through image processing. The fluorescence intensity $I_i$ of the bright spots of the evaluation dye and the number $N$ of bright spots (first bright spot number of bright spots included in the fluorescence image 2004) are acquired (extracted) from the evaluation fluorescence image 2004. The number $N'$ of bright spots (second bright spot number of spots included in the fluorescence image 2009) is acquired (extracted) from the reference fluorescence image 2009.

The method for extracting bright spots is not particularly limited and can be implemented by a general image processing method. Since a reference dye is sufficiently bright, the number of bright spots included in the reference fluorescence image 2009 indicates the number of particles existing in the image. Bright spots included in the evaluation fluorescence image 2004 indicate particles that emit fluorescent light beyond the detection limit of the fluorescence measurement apparatus 1000. Thus, as expressed by equation (6) below, the ratio of detected particles (the detection rate r) is calculated using a ratio of the bright spot number $N$ to the bright spot number $N'$:

$$r = \frac{N}{N'} \qquad \ldots (6)$$

The average value $\bar{I}$ the fluorescence intensity of the evaluation dye is predicted using the obtained detection rate r. It is considered that the fluorescence intensity $I_i$ measured for each particle follows a probability density function $f(I)$. The detection rate is a ratio of particles for which the fluorescence intensity $I_i$ is equal to or larger than a detection limit $I_{min}$ of the fluorescence measurement apparatus 1000. Thus, equation (7) below holds with a cumulative distribution function $F(I)$ for $I$:

$$r = 1 - F(I_{min}) \qquad \ldots (7)$$

The probability density function $f(I)$ and its cumulative distribution function are typically defined by using a plurality of coefficients, but a constraint condition can be imposed on the plurality of coefficients by equation (7).

The probability density function $f(I)$ of the measured fluorescence intensity $I_i$ may be well reproduced by a log-normal distribution (log-normal distribution function). That is, f(I) can be expressed by equation (8) below:

$$f(I) = \frac{1}{\sqrt{2\pi\sigma^2}I} \exp\left(-\frac{(\ln I - \mu)^2}{2\sigma^2}\right) \quad \cdots (8)$$

Coefficients $\mu$ and $\sigma$ are coefficients (coefficients associated with the detection rate r) that determine the log-normal distribution. Using the cumulative distribution function (log-normal distribution function) for the log-normal distribution can provide a constraint condition on the coefficients $\mu$ and $\sigma$ from equation (7), as expressed by equation (9) below:

$$r = \frac{1}{2} - \frac{1}{2}\mathrm{erf}\left(\frac{\ln I_{min} - \mu}{\sigma\sqrt{2}}\right) \quad \cdots (9)$$

where erf is an error function.

The coefficients $\mu$ and $\sigma$ may be estimated from this constraint condition and the fluorescence intensity $I_i$. It is possible to obtain $I_{min}$ as the minimum value of $I_i$. From equation (9), $\mu$ and $\sigma$ have a one-to-one relation. Accordingly, only one of the coefficients $\mu$ and $\sigma$ is to be calculated. Thus, $\mu$ and $\sigma$ can be obtained by calculating f(I), which best reproduces the histogram (fluorescence amount) of $I_i$, by fitting under the constraint condition of equation (9).

The average value of the log-normal distribution is expressed as equation (10) below by using the coefficients $\mu$ and $\sigma$:

$$\bar{I} = e^{\mu + \frac{\sigma^2}{2}} \quad \cdots (10)$$

The average value $\bar{I}$ of the fluorescence intensity of the calibration particles (statistical value of the fluorescence amount) can be calculated (predicted) from the coefficients $\mu$ and $\sigma$ obtained by equation (10) and fitting. In equation (10), the average value is calculated from the obtained coefficients $\mu$ and $\sigma$, but the average value may be calculated by using the probability density function f(I) determined from these coefficients.

Each embodiment predicts coefficients that determine the probability density function f(I), or the probability density function f(I) itself based on the detection rate r, and calculates the average value $\bar{I}$ of the fluorescence intensity based on the predicted coefficients or the probability density function f(I). Thereby, even when only the fluorescence intensities of limited particles are known, the average value $\bar{I}$ of the fluorescence intensity for all particles can be correctly predicted as compared to calculating the average value $\bar{I}$ from only the measured fluorescence intensity $I_i$.

Once the correct average value $\bar{I}$ is calculated, the correct conversion coefficient $\gamma$ can be found by equation (4). Using the correct conversion coefficient $\gamma$ can correctly estimate the dye amount of each specimen by equation (5).

By using a known number $\beta$ of dyes that a ligand such as an antibody that is used when a dye is bound to the specimen contains per ligand, the amount $y_i$ of expressed protein per specimen can be estimated, as expressed by equation (11) below:

$$y_i = \frac{x_i}{\beta} \quad \cdots (11)$$

In each embodiment, the fluorescence measurement apparatus 1000 can be evaluated as well. The minimum fluorescence intensity detectable by the fluorescence measurement apparatus 1000, that is, the detection limit $I_{min}$ for the fluorescence intensity is the minimum value of $I_i$. Using the conversion coefficient $\gamma$ calculated as described above can estimate, from $I_{min}$, the minimum number of fluorescent dyes per specimen that the fluorescence measurement apparatus 1000 needs for detection, that is, a detection limit $x_{min}$ of the number of dyes.

$$x_{min} = \frac{I_{min}}{\gamma} \quad \cdots (12)$$

In other words, the detection limit of the number of dyes means that, as long as one specimen has a number of dyes equal to or larger than the detection limit $x_{min}$ of the number of dyes, the specimen can be detected from the fluorescence image 2004. Evaluating $x_{min}$ before the specimen is measured can ensure that the detected specimen has fluorescent dyes equal to or larger than $x_{min}$. By evaluating $x_{min}$ over time, it is possible not only to recognize the degradation status and maintenance timing of the fluorescence measurement apparatus 1000 but also to evaluate the performance of the fluorescence measurement apparatus 1000 by comparing $x_{min}$ between fluorescence measurement apparatuses 1000.

Using the known number $\beta$ of dyes that a ligand used to bind a dye to the specimen has per ligand can calculate a detection limit $y_{min}$ of the amount $y_i$ of protein expressed in the specimen, as expressed by equation (13) below:

$$y_{min} = \frac{x_{min}}{\beta} \quad \cdots (13)$$

In other words, the detection limit of the amount of protein means that, if one specimen expresses an amount of protein equal to or larger than the detection limit $y_{min}$ of the amount of protein, the specimen can be detected from the fluorescence image 2004. Evaluating the detection limit $y_{min}$ before the specimen is measured can ensure that the detected specimen expresses an amount of protein equal to or larger than $y_{min}$.

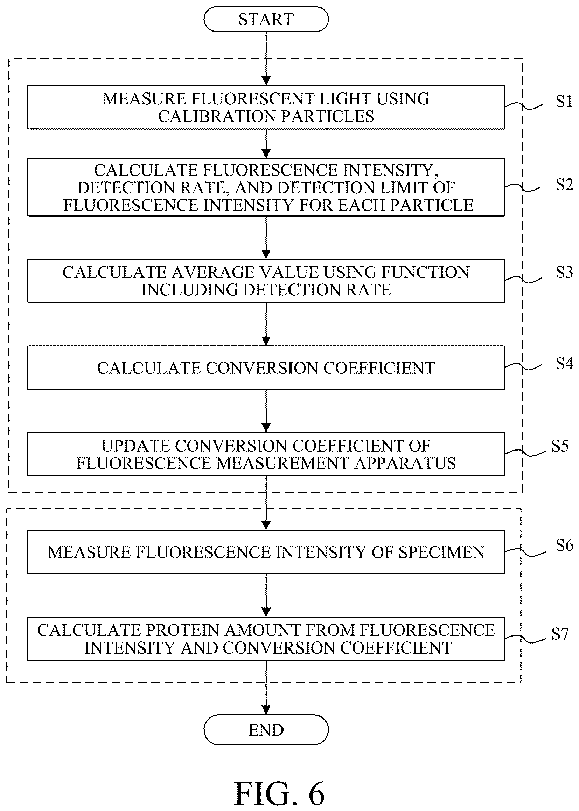

The above calibration method (measurement method) according to each embodiment will be described below with reference to Fig. 6. Fig. 6 is a flowchart illustrating the measurement method according to each embodiment.

First, in step S1, the fluorescence measurement apparatus 1000 (control unit 1300) acquires fluorescence images using the calibration particles. The calibration particles are immobilized on the substrate 1400, and the fluorescence images 2004 and 2009 are acquired by using the fluorescence measurement apparatus 1000 along with a light source and a fluorescence filter with a wavelength corresponding to each dye.

Next, in step S2, the control unit 1300 calculates, through image processing, the fluorescence intensity $I_i$ and the bright spot number N for an evaluation fluorescent dye from the evaluation fluorescence image 2004 acquired at S1, and the bright spot number N' from the reference fluorescence image 2009. Then, the control unit 1300 calculates the detection rate r using the bright spot numbers N and N' and equation (6). In addition, the control unit 1300 calculates the detection limit $I_{min}$ of the fluorescence intensity from the minimum value of $I_i$.

Next, in step S3, the control unit 1300 calculates the average value $\bar{I}$ of the fluorescence intensity from $I_i$ using a probability density function including the detection rate r. The probability density function including the detection rate is a probability density function in which a constraint condition is imposed on a plurality of coefficients that determine the probability density function by equation (7) or (9). The control unit 1300 calculates, by fitting, the coefficient $\mu$ or $\sigma$ for reproducing the histogram of $I_i$ under the constraint condition, and calculates the average value $\bar{I}$ of the fluorescence intensity from the obtained coefficient based on equation (10).

Next in step S4, the control unit 1300 calculates the conversion coefficient $\gamma$ from the obtained average value $\bar{I}$ of the fluorescence intensity. The conversion coefficient $\gamma$ can be calculated by using equation (4). The average number of dyes $\bar{x}$ for equation (4) can be calculated from measured values of the absorbance and particle concentration of the calibration particles.

Next, in step S5, the control unit 1300 updates a conversion coefficient held (stored) in the fluorescence measurement apparatus 1000 to the conversion coefficient calculated in steps S1 to S4. The conversion coefficient may be updated by inputting a calculation result into the fluorescence measurement apparatus 1000 by a user through an input device or may be automatically updated by the fluorescence measurement apparatus 1000.

The above steps S1 to S5 constitute the calibration step. Since the sensitivity of the fluorescence measurement apparatus 1000 changes due to the deterioration over time and external environment changes, the calibration step is performed periodically or for each measurement.

Next, in step S6, the control unit 1300 measures the fluorescence intensity of the specimen. The specimen labelled with an evaluation fluorescent dye is immobilized on the substrate 1400, and fluorescence images are acquired. The control unit 1300 performs image processing for the obtained images, thereby extracting the fluorescence intensity $I_i$ of bright spots.

Next, in step S7, using the extracted fluorescence intensity $I_i$, the conversion coefficient $\gamma$ updated at the calibration step, and equations (5) and (11), the control unit 1300 acquires (calculates) the number of dyes $x_i$ and the amount $y_i$ of protein in each specimen.

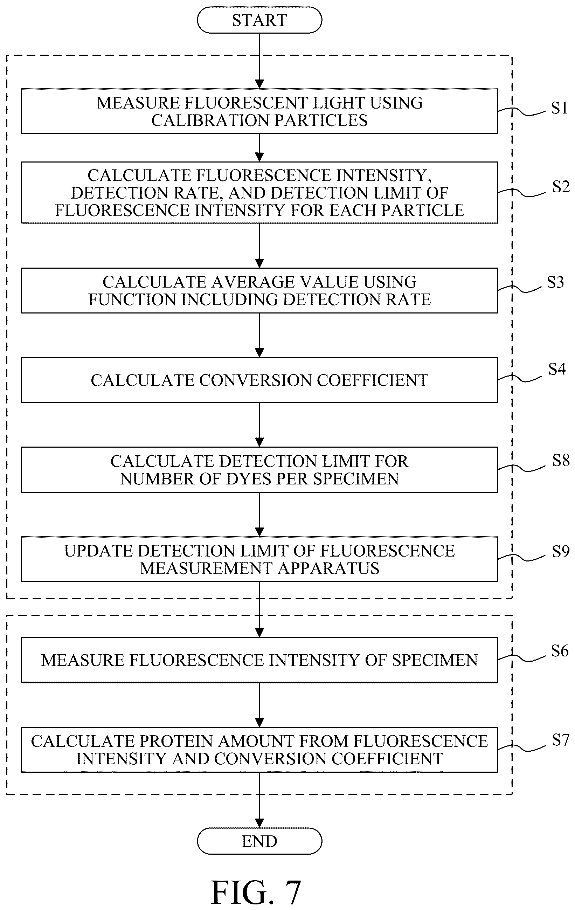

In a case where each embodiment is used to evaluate the detection limit of the fluorescence measurement apparatus 1000, a flowchart illustrated in Fig. 7 is used. Fig. 7 is a flowchart illustrating another measurement method according to each embodiment. Fig. 7 is different from Fig. 6 in steps S8 and S9 provided in place of steps S4 and S5. The following description will omit a common description to that of Fig. 6.

In step S8, the control unit 1300 calculates the detection limit $x_{min}$ of the number of dyes per specimen from the detection limit $I_{min}$ of the fluorescence intensity and the conversion coefficient y, which are calculated through the processing in steps S1 to S4, based on equation (12). That is, the control unit 1300 acquires the minimum value (detection limit $x_{min}$) of a number of dyes that the fluorescence measurement apparatus 1000 can measure, using the conversion coefficient $\gamma$ and the minimum value (detection limit $I_{min}$) of a fluorescence amount (fluorescence intensity) that the fluorescence measurement apparatus 1000 can measure.

Next, in step S9, the control unit 1300 updates the value of the detection limit $x_{min}$ of the number of dyes, which is recorded in the fluorescence measurement apparatus 1000, to the value calculated in step S8. The conversion coefficient may be updated by inputting a calculation result into the fluorescence measurement apparatus 1000 by a user through an input device or may be automatically updated by the fluorescence measurement apparatus 1000. The value calculated in step S8 and updated in step S9 is not limited to the detection limit $x_{min}$ of the number of dyes but may be the detection limit $y_{min}$ of the amount of protein (the minimum value of the amount of protein that the fluorescence measurement apparatus 1000 can measure).

Each embodiment can calculate the number $x_i$ of dyes and the amount $y_i$ of protein in the specimen with high accuracy. Each embodiment can evaluate the detection limit of the fluorescence measurement apparatus with high accuracy.

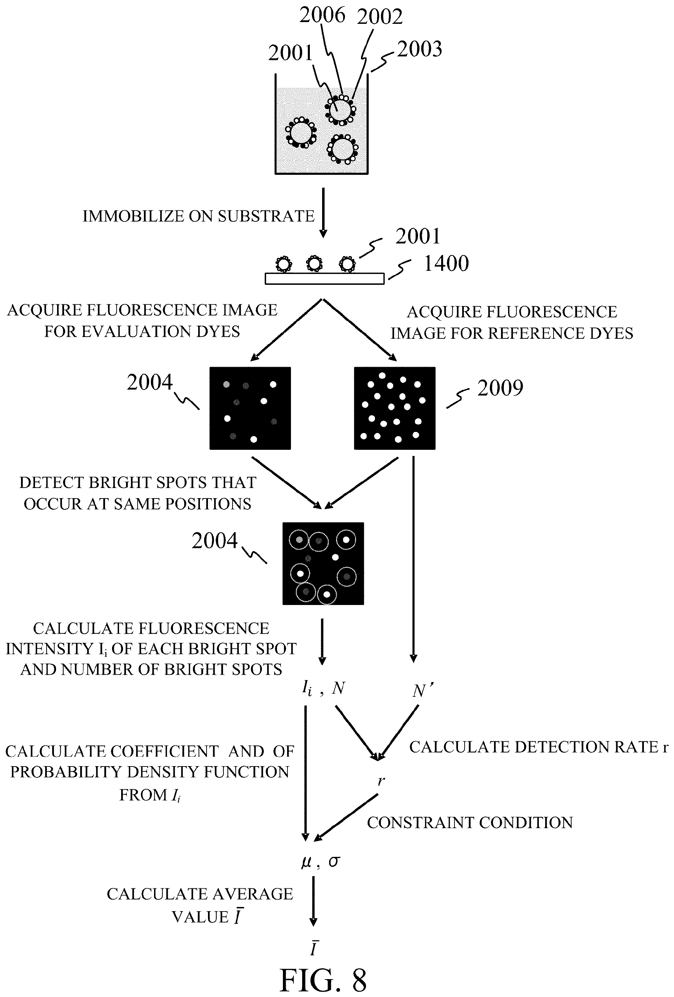

A description will be given of a variation of each embodiment with reference to Fig. 8. Fig. 8 is a schematic view illustrating a measurement method according to a variation of each embodiment. As illustrated in Fig. 8, in this variation, in detecting bright spots from the fluorescence image 2004, the two fluorescence images 2004 and 2009 are compared to specifying bright spots generated at the same positions.

Since light from the evaluation dye is weak, unexpected light emission from impurities or noise generated in the image sensor may be mixed in the fluorescence image 2004. Such impurities or noise are hardly observed at approximately the same positions in the two images, and thus it is possible to remove unnecessary signals by specifying only bright spots generated at approximately identical places in the two images. Approximately the same position does not mean exactly the same position, but positions can be regarded as the same if the coordinates match within a certain range, for example, within several times the size of the point spread function of the objective lens 1101 or within a few pixels. Highly accurate measurement with reduced unnecessary signal influence can be performed by calculating the fluorescence intensity $I_i$ and the bright spot number N for bright spots specified as the same positions and calculating the average value $\bar{I}$ similarly to the method described above with reference to Fig. 2.

Each embodiment has discussed the log-normal distribution as the probability density function, but is not limited to this example. For example, gamma distribution, beta distribution, and Weibull distribution are known as functions similar to the log-normal distribution and may be used as f(I) for analysis. The log-normal distribution best represents the frequency of the fluorescence intensity $I_i$ and is easy to manage, and thus may be used as the probability density function.

In each embodiment, the above method for calculating coefficients for the probability density function uses fitting, but is not limited to this example. In the log-normal distribution, the ratio of the average value $\bar{I}$ and a standard deviation s has a relation expressed by equation (14) below:

$$\frac{s}{\bar{I}} = \sqrt{e^{\sigma^2} - 1} \qquad \ldots (14)$$

This ratio is determined by the produced particles and thus does not depend on the fluorescence measurement apparatus 1000. Thus, separately from the fluorescence measurement apparatus 1000 to be evaluated, a fluorescence measurement apparatus having a higher sensitivity than the fluorescence measurement apparatus 1000 may be used to previously calculate this ratio. Thereby, the coefficient $\sigma$ can be calculated from equation (14). The coefficient $\mu$ can be calculated from equation (9), and the coefficients can be calculated without using fitting.

This method is not limited to the log-normal distribution but may be performed similarly for another probability density function. Two coefficients for determining the gamma distribution or beta distribution can be determined from the two constraint conditions, namely a constraint condition on the average value and the standard deviation and a constraint condition on the detection rate corresponding to equation (9).

Each example is also applicable to evaluation of the calibration particles. Different calibration particles can be measured by the same fluorescence measurement apparatus 1000, and the value of $\bar{I}$, y, or $x_{min}$ is compared among the particles to evaluate their performance.

The description in each embodiment does not particularly distinguish the calibration particles for obtaining the conversion coefficient $\gamma$ and the calibration particles for evaluating the detection limit of the fluorescence measurement apparatus, but the calibration particles may be different in accordance with an application. Since the conversion coefficient $\gamma$ is used to measure protein in the specimen, the conversion coefficient $\gamma$ may be measured by using calibration particles that simulate biochemical characteristics of the specimen to be actually measured. For example, in a case where the specimen is a vesicle such as an extracellular vesicle or a virus, the refractive index is close to that of water, and particles to which evaluation fluorescent dyes are bound on the surface via an antibody may be used as the calibration particles. The particles to be bound may be particles with a low refractive index, such as silica particles, or vesicles such as liposomes.

On the other hand, since the fluorescence measurement apparatus 1000 is used for measurement of a variety of specimens, it is sufficient to use particles that simulate physical characteristics of general specimens in evaluating the detection limit of the fluorescence measurement apparatus. For advantages in easy handling and general-purpose applicability, particles may be chemically combinable. Since a biologically derived specimen generally has a low refractive index, particles such as silica may be used. Examples of methods for binding the fluorescent dye include amide bonding, which is chemically easy to process.

While each embodiment illustrates a method for measuring the amount of protein, the disclosure is not limited to this embodiment. For example, the amount of DNA or RNA expressed in a specimen may be measured.

Each example will be specifically described below.

FIRST EMBODIMENT

A first embodiment will be now described. The fluorescence measurement apparatus 1000 according to this embodiment illustrated in Fig. 1 includes, as the light source 1201, two light sources: an LED having a central wavelength of 475 nm and an LED having a central wavelength of 630 nm.

As the filter cube (fluorescence filter) 1204, a fluorescence filter set 1 for observing a green fluorescent dye and a fluorescence filter set 2 for observing a red fluorescent dye are prepared in a turret. The fluorescence filter set 1 includes an excitation filter having a central wavelength of 480 nm and a bandwidth of 30 nm, a long-path dichroic mirror having a cut-on wavelength of 505 nm, and an absorption filter having a central wavelength of 535 nm and a bandwidth of 40 nm. The fluorescence filter set 2 includes an excitation filter having a central wavelength of 620 nm and a bandwidth of 50 nm, a long-path dichroic mirror having a cut-on wavelength of 655 nm, and an absorption filter having a central wavelength of 690 nm and a bandwidth of 50 nm.

The illumination unit 1200 includes the general collimator lens 1202 and condenser lens 1203, and constitutes a Kohler illumination system together with the objective lens 1101. The objective lens 1101 has a magnification of 40 and a NA of 0.95. Fluorescence images of the sample 1500 immobilized on the substrate 1400 are captured on the image sensor (CMOS sensor) 1103 through the objective lens 1101, the filter cube 1204, and the imaging lens 1102.

The calibration particles are produced by binding to silica particles, a red cyanine-based fluorescent dye having an NHS ester at the terminal as an evaluation fluorescent dye and a green fluorescein-based fluorescent dye having an NHS Ester at the terminal as a reference fluorescent dye. The silica particles have a diameter of 100 nm approximately and have amino groups on their surfaces. A sufficient amount of the green reference fluorescent dye is bound to the silica particles. An amount of the red evaluation fluorescent dye comparable to that bound to the specimen is bound. The calibration particles are immobilized by dropping a solution in which the produced calibration particles are dispersed onto a glass substrate and drying the solution. Fluorescence images of the immobilized particles are acquired by using a light source and a fluorescence filter corresponding to each fluorescent dye.

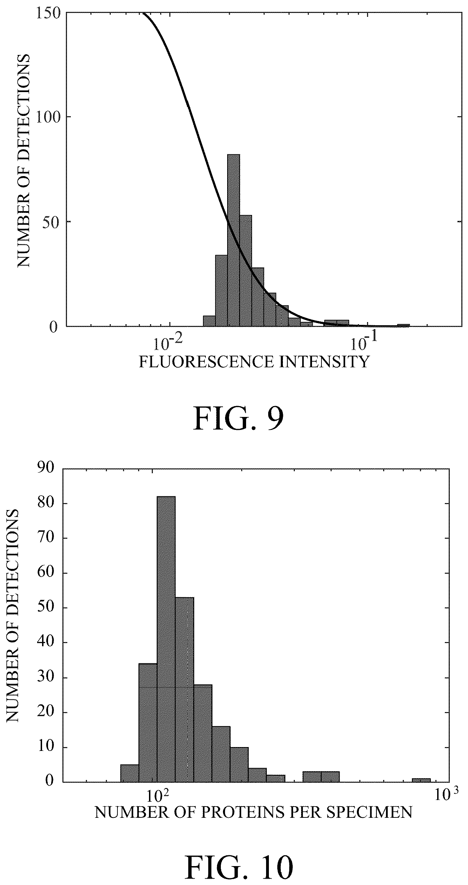

Image processing is performed for two obtained fluorescence images to calculate the detection rate r and the fluorescence intensity $I_i$ of bright spots included in the evaluation fluorescence image 2004. A value obtained by normalizing an output value from the image sensor (CMOS sensor) 1103 with respect to the saturated value of each pixel is used as the fluorescence intensity. The detection rate r thus calculated is 9.1%, and the detected fluorescence intensity is a histogram illustrated in Fig. 9. Fig. 9 illustrates a measurement result in this embodiment. In Fig. 9, the horizontal axis represents the fluorescence intensity, and the vertical axis represents the number of detections.

The log-normal distribution expressed by equation (8) is assumed as the probability density function, and $\mu$ and $\sigma$ that best reproduce the histogram of $I_i$ are calculated while the constraint condition in equation (9) is imposed. An estimated function is illustrated as a straight line in Fig. 9. The average value $\bar{l}$ calculated from the coefficients $\mu$ and $\sigma$ based on equation (10) is 0.0087. A simple average value of the measured fluorescence intensities $I_i$ is 0.0259. In a case where the detection rate is considered from the ratio of both numbers, the fluorescence intensity can be corrected about three times as compared to a case where the detection rate is not considered.

The fluorescence measurement apparatus 1000 may include a dark-field observation optical system. In a case where particles illustrated in Fig. 4C or 4A are used as the calibration particles 2001, a dark-field observation image may be used as the reference fluorescence image 2009 to calculate the detection rate r.

SECOND EMBODIMENT

A second embodiment will be described next. This embodiment calculates the conversion coefficient $\gamma$ from the average value $\bar{l}$ of the fluorescence intensity calculated in the first embodiment. The absorption spectrum of a solution containing produced calibration particles is measured by a spectrophotometer. The absorbance A at the wavelength of 656 nm where the absorption of the evaluation dye has a peak is evaluated to be A = 0.18. The molar absorption coefficient of the evaluation dye is $\varepsilon$ = 239000 (cm$^{-1}$M$^{-1}$), and the evaluation dye concentration $c_A$ is calculated to be $7.5 \times 10^{-7}$ (M) from the thickness L = 1 cm of a cell filled with the solution based on equation (2).

The absorbance of a dispersion liquid of silica particles having a known particle number concentration and the same diameter is measured by the same spectrophotometer and compared with that of the solution of the calibration particles, and thereby the particle number concentration is measured. From the obtained comparison result, the concentration $c_p$ of the calibration particles was obtained as $3.2 \times 10^{-9}$ (M). From the obtained measurement result, the average value $\bar{x_0}$ of the number of dyes bound to the calibration particles is calculated to be 231 based on equation (3). Using the average value $\bar{l}$ = 0.0087 predicted in the first embodiment and equation (4) can provide the conversion coefficient $\gamma$ as $3.8 \times 10^{-5}$. Fig. 10 illustrates a measurement result in this embodiment, which is a result obtained by converting the fluorescence intensity $I_i$ into the number of proteins per specimen based on the obtained conversion coefficient y, the number of dyes $\beta$ bound per ligand, and equations (5) and (11). In Fig. 10, the horizontal axis represents the number of proteins per specimen, and the vertical axis represents the number of detections. The number of dyes $\beta$ (number of bindings) is set to five, which is a general value for the number of bindings between a red evaluation fluorescent dye and an antibody. This embodiment can determine proteins bound to each specimen and its distribution. In addition, more detailed analysis of the specimen is possible by analyzing statistical properties such as an average value, a standard deviation, and a distribution shape from the result illustrated in Fig. 10.

THIRD EMBODIMENT

A third embodiment will be described next. This embodiment calculates the detection limit $x_{min}$ of the number of dyes from the detection limit $I_{min}$ of the fluorescence intensity calculated from the measurement result illustrated in Fig. 9, and the conversion coefficient $\gamma$ calculated in second embodiment. $I_{min}$ is 0.018 from the minimum value of the fluorescence intensity $I_i$ calculated

in first embodiment. From the conversion coefficient $\gamma = 3.8 \times 10^{-5}$ obtained in the second embodiment and equation (12), the detection limit $x_{min}$ of the number of dyes of the fluorescence measurement apparatus 1000 is calculated to be 470.

In a case where the number of dyes $\beta$ bound per ligand is assumed to be the general value of five, the detection limit $y_{min}$ of the protein number of the fluorescence measurement apparatus 1000 is calculated to be 94 from equation (13).

FOURTH EMBODIMENT

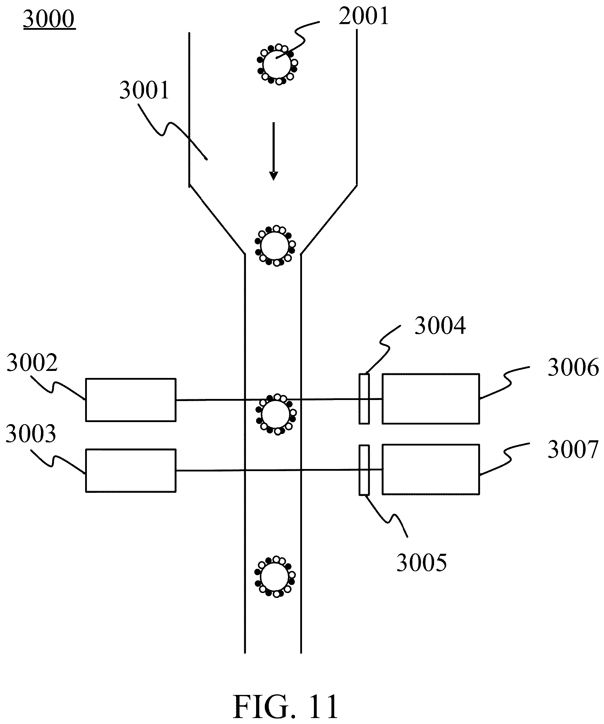

A fourth embodiment will be described next. This embodiment uses a flow cytometer 3000 as the fluorescence measurement apparatus. Fig. 11 is a schematic diagram of the flow cytometer (measurement apparatus) 3000 in this embodiment.

The flow cytometer 3000 emits light from laser beam sources 3002 and 3003 to each calibration particle 2001 flowing inside a flow path 3001. Fluorescent light emitted from the calibration particle 2001 is detected by photomultipliers 3006 and 3007 through band-pass filters 3004 and 3005. The flow path 3001 has such a narrow flow path that only one particle flows in a laser irradiation region. The laser beam source 3002 is a semiconductor laser having a wavelength of 488 nm. The laser beam source 3003 is a semiconductor laser having a wavelength of 635 nm. The band-pass filter 3004 has a central wavelength of 525 nm and a bandwidth 50 nm. The band-pass filter 3005 has a central wavelength of 700 nm and a bandwidth 50 nm.

The calibration particles are the same as those of first embodiment. The calibration particles are flowed at such a flow rate that it can be determined that fluorescent light from of the evaluation dye and a signal of the reference dye are emitted from the same particle in time, and each fluorescence intensity is measured. In this embodiment, current values output from the photomultipliers 3006 and 3007 serve as the fluorescence intensities. The number of observations (second bright spot number) of the reference fluorescent light is set as N', the number of measurements (first bright spot number) of evaluation fluorescent light is set as N, and the detection rate r is measured based on equation (6). The average value of the fluorescence intensity can be calculated with high accuracy by performing calculation similarly to first embodiment by using the detection rate r and the evaluation fluorescence intensity $I_i$.

The flow cytometer 3000 may include a photomultiplier for measuring side scattering. In a case where particles illustrated in Fig. 4C or 4A are used as the calibration particles 2001, side-scattered light can be used as the reference light. The wavelength characteristics of the laser beam sources 3002 and 3003 and the band-pass filters 3004 and 3005 are properly changed in accordance with a fluorescent dye to be evaluated.

Each embodiment has discussed a calibration method that calculates the average value $\bar{I}$ from measured $I_i$, but is not limited to this example. For example, the median of $I_i$ may be used. Since the absorbance A is an amount measured for the entire particle solution, the average value $\bar{I}$ has a good correspondence relationship with the average value $\bar{x}$ of the number of particles, which is calculated from the absorbance. Thus, the average value $\bar{I}$ may be used.

OTHER EMBODIMENTS

Embodiment(s) of the disclosure can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)TM), a flash memory device, a memory card, and the like.

Each embodiment can provide a measurement method that can accurately measure weak fluorescent light.

**Claims**

1. A measurement method comprising:

   measuring fluorescent light emitted from fluorescent dyes (2002) of each of a plurality of fine particles (2001) that are bound with the fluorescent dyes (2002), and measuring reference light emitted from each of the plurality of fine particles (2001);
   acquiring a ratio (r) of particles for which both the fluorescent light from the fluorescent dyes (2002) and the reference light are detected in the plurality of fine particles (2001); and
acquiring a statistical value of a fluorescence amount of the fluorescent dyes (2002) based on the fluorescence amount of the fluorescent dyes (2002) and the ratio of the particles.

2. The measurement method according to claim 1, **characterized in that** acquiring the statistical value acquires the statistical value using a function including the ratio (r) of the particles.

3. The measurement method according to claim 2, **characterized in that** the function is a log-normal distribution function including a coefficient associated with the ratio of the particles.

4. The measurement method according to any one of claims 1 to 3, further comprising:
acquiring a conversion coefficient corresponding to a fluorescence amount of the fluorescent dyes (2002) per dye by using the statistical value and an average value of the number of the fluorescent dyes bound to the fine particles (2001).

5. The measurement method according to claim 4, further comprising:
calculating a dye amount of a specimen bound to the fluorescent dyes (2002) via ligands, using the conversion coefficient and a fluorescence amount of the specimen.

6. The measurement method according to claim 5, further comprising:
acquiring an amount of protein of the specimen by using the number of dyes of the fluorescent dyes (2002) relative to one of the ligands and the dye amount.

7. The measurement method according to claim 4, further comprising:
acquiring a minimum amount of the number of dyes indicating a measurement limit of a measurement apparatus (1000), by using the conversion coefficient and a minimum value of the fluorescence amount of the fluorescent dyes (2002) indicating the measurement limit of the measurement apparatus (1000).

8. The measurement method according to claim 7, further comprising:
calculating a minimum amount of protein indicating the measurement limit of the measurement apparatus (1000) by using the minimum value.

9. The measurement method according to any one of claims 4 to 6, further comprising updating the conversion coefficient stored in a measurement apparatus (1000) by using the conversion coefficient.

10. The measurement method according to any one of claims 1 to 9, **characterized in that** acquiring the ratio includes:

immobilizing the plurality of fine particles (2001) on a substrate (1400),
acquiring a first image (2004) of the plurality of fine particles (2001) for the fluorescent dyes and a second image (2009) of the plurality of fine particles (2001) for the reference light,
acquiring a first number (N) of bright spots located at the same position in the first image and the second image and a second number (N') of bright spots included in the second image, and
acquiring the ratio (r) of the particles by acquiring a ratio of the first number of bright spots to the second number of bright spots.

11. The measurement method according to any one of claims 1 to 10, **characterized in that** the statistical value is an average value.

12. The measurement method according to any one of claims 1 to 11, further comprising:
measuring fluorescent light of a specimen labeled with fluorescent dyes that are the same as the fluorescent dyes (2002) bound to the plurality of fine particles (2001).

13. The measurement method according to any one of claims 1 to 12, **characterized in that** the fine particles (2001) have diameters of 10 nm to 500 nm.

14. The measurement method according to any one of claims 1 to 13, **characterized in that** the fine particles (2001) include silica or polystyrene.

15. The measurement method according to any one of claims 1 to 14, **characterized in that** the reference light is scattered light from the fine particles (2001) or fluorescent light from fluorescent dyes (2006) different from the fluorescent dyes (2002) bound on the fine particles (2001).

16. The measurement method according to any one of claims 1 to 15, **characterized in that** a wavelength of the reference light is shorter than a wavelength of light from the fluorescent dyes.

17. The measurement method according to any one of claims 1 to 16, **characterized in that** the fine particles (2001) include extracellular vesicles or viruses.

18. The measurement method according to any one of claims 1 to 17, **characterized in that** measuring the fluorescent light and the reference light includes immobilizing the fine particles (2001) on a plasmonic substrate.

19. A measurement apparatus (1000) comprising:

a measurement unit (1100, 1200) configured to measure fluorescent light emitted from fluorescent dyes (2002) of each of a plurality of fine particles (2001) that are bound with the fluorescent dyes, and configured to measure reference light emitted from each of the plurality of fine particles;
a first acquiring unit (1301) configured to acquire a ratio of particles for which both the fluorescent light from the fluorescent dyes (2002) and the reference light are detected in the plurality of fine particles (2001); and
a second acquiring unit (1302) configured to acquire a statistical value of a fluorescence amount of the fluorescent dyes (2002) based on the fluorescence amount of the fluorescent dyes and the ratio of the particles.

20. A program that when executed on a computer causes the computer to perform the measurement method according to any one of claims 1 to 18.

1000

1300

1301

1302

1100

1103

1102

1200

1204

1101

1203

1202

1201

1500

1400

FIG. 1

FIG. 2

FIG. 3

FIG. 4A

FIG. 4B

FIG. 4C

FIG. 4D

2006 2002

2001 2003

IMMOBILIZE ON
SUBSTRATE

2001
1400

ACQUIRE
FLUORESCENCE IMAGE
FOR EVALUATION DYES

ACQUIRE
FLUORESCENCE IMAGE
FOR REFERENCE DYES

2004

2009

CALCULATE FLUORESCENCE
INTENSITY $I_i$ OF EACH
BRIGHT SPOT AND NUMBER
OF BRIGHT SPOTS

$I_i$ , $N$

$N'$

CALCULATE DETECTION RATE r

CALCULATE COEFFICIENT
AND OF PROBABILITY
DENSITY FUNCTION FROM $I_i$

$r$

CONSTRAINT CONDITION

$\mu$ , $\sigma$

CALCULATE AVERAGE
VALUE $\bar{I}$

$\bar{I}$

# FIG. 5

START

MEASURE FLUORESCENT LIGHT USING
CALIBRATION PARTICLES — S1

CALCULATE FLUORESCENCE INTENSITY,
DETECTION RATE, AND DETECTION LIMIT OF
FLUORESCENCE INTENSITY FOR EACH PARTICLE — S2

CALCULATE AVERAGE VALUE USING FUNCTION
INCLUDING DETECTION RATE — S3

CALCULATE CONVERSION COEFFICIENT — S4

UPDATE CONVERSION COEFFICIENT OF
FLUORESCENCE MEASUREMENT APPARATUS — S5

MEASURE FLUORESCENCE INTENSITY OF SPECIMEN — S6

CALCULATE PROTEIN AMOUNT FROM FLUORESCENCE
INTENSITY AND CONVERSION COEFFICIENT — S7

END

FIG. 6

START

MEASURE FLUORESCENT LIGHT USING
CALIBRATION PARTICLES — S1

CALCULATE FLUORESCENCE INTENSITY,
DETECTION RATE, AND DETECTION LIMIT OF — S2
FLUORESCENCE INTENSITY FOR EACH PARTICLE

CALCULATE AVERAGE VALUE USING
FUNCTION INCLUDING DETECTION RATE — S3

CALCULATE CONVERSION COEFFICIENT — S4

CALCULATE DETECTION LIMIT FOR
NUMBER OF DYES PER SPECIMEN — S8

UPDATE DETECTION LIMIT OF FLUORESCENCE
MEASUREMENT APPARATUS — S9

MEASURE FLUORESCENCE INTENSITY OF SPECIMEN — S6

CALCULATE PROTEIN AMOUNT FROM FLUORESCENCE
INTENSITY AND CONVERSION COEFFICIENT — S7

END

FIG. 7

2006 2002

2001 2003

IMMOBILIZE ON SUBSTRATE

2001

1400

ACQUIRE FLUORESCENCE IMAGE
FOR EVALUATION DYES

ACQUIRE FLUORESCENCE
IMAGE FOR REFERENCE DYES

2004

2009

DETECT BRIGHT SPOTS THAT
OCCUR AT SAME POSITIONS

2004

CALCULATE FLUORESCENCE
INTENSITY $I_i$ OF EACH BRIGHT SPOT
AND NUMBER OF BRIGHT SPOTS

$I_i$ , $N$          $N'$

CALCULATE COEFFICIENT AND OF
PROBABILITY DENSITY FUNCTION
FROM $I_i$

CALCULATE DETECTION RATE r

$r$

CONSTRAINT CONDITION

$\mu$ , $\sigma$

CALCULATE AVERAGE
VALUE $\bar{I}$

$\bar{I}$

## FIG. 8

FIG. 9

FIG. 10

FIG. 11

Europäisches
Patentamt
European
Patent Office
Office européen
des brevets

# EUROPEAN SEARCH REPORT

**Application Number**

EP 25 21 7456

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/123746 A1 (IM HYUNGSOON [US] ET AL) 20 April 2023 (2023-04-20) * paragraphs [0006] - [0276]; figures 1A-24B * | 1-20 | INV. G01N15/0227 G01N15/1429 G01N15/1434 G01N21/552 |
| X | US 2024/310367 A1 (HUANG XIAOHUA [US] ET AL) 19 September 2024 (2024-09-19) * paragraphs [0006] - [0127]; figures 1-12 * | 1-20 | G01N21/64 G01N33/50 G01N33/58 |

**TECHNICAL FIELDS SEARCHED (IPC)**

G01N

The present search report has been drawn up for all claims

| Place of search | Date of completion of the search | Examiner |
|---|---|---|
| The Hague | 27 March 2026 | Ionescu, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
........................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 21 7456

This annex lists the patent family members relating to the patent documents cited in the above-mentioned European search report.
The members are as contained in the European Patent Office EDP file on
The European Patent Office is in no way liable for these particulars which are merely given for the purpose of information.

27-03-2026

| Patent document cited in search report | Publication date | Patent family member(s) | Publication date |
|---|---|---|---|
| US 2023123746 A1 | 20-04-2023 | NONE | |
| US 2024310367 A1 | 19-09-2024 | US 2020191778 A1 | 18-06-2020 |
| | | US 2024310367 A1 | 19-09-2024 |
| | | WO 2020132074 A1 | 25-06-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023521872 A

**Non-patent literature cited in the description**

- **JEONG, MI HO et al.** Plasmon-Enhanced Single Extracellular Vesicle Analysis for Cholangiocarcinoma Diagnosis.. *Advanced Science*, January 2023, vol. 10 (8), 2205148